# EUROPEAN PATENT APPLICATION

(11) **EP 4 813 299 A1**
(43) Date of publication of application: **30.09.2026**
(21) Application number: 24892915.0
(22) Date of filing: 15.08.2024
(51) Int. Cl.: A61B 6/00

(54) **TOMOSYNTHESIS IMAGING DEVICE AND METHOD FOR TARGET REGION**

(30) Priority: 21.11.2023 CN 202311553778
(71) Applicant: Nuray Technology Co., Ltd., Changzhou, Jiangsu 213200 (CN)
(72) Inventor: JIN, Xin, Beijing 100084 (CN); TANG, Huaping, hangzhou, Jiangsu 213200 (CN); XIE, Dehua, hangzhou, Jiangsu 213200 (CN); FANG, Runze, hangzhou, Jiangsu 213200 (CN)
(74) Representative: Dilg, Haeusler, Schindelmann Patentanwaltsgesellschaft mbH
(86) International application number: PCT/CN2024/112398
(87) International publication number: WO 2025/107753

(57) **Abstract**

The present disclosure provides a tomosynthesis imaging apparatus and method. The apparatus includes: a distributed multi-point emission radiation source having an array of a plurality of point sources, where the plurality of point sources are configured to sequentially emit radiation beams; and a detector configured to be placed inside the target region (such as an oral cavity) or any other suitable position relative to the target region to detect the radiation beams emitted by the distributed multi-point emission radiation source. The detector is configured to start an acquisition of the radiation beams upon a detection of an increase in an intensity of the radiation beams, and to stop the acquisition upon a detection of a decrease in the intensity of the radiation beams. The detector of the present disclosure does not need to be in communicative connection with a controller or processor of the distributed multi-point emission radiation source.

## Description

### TECHNICAL FIELD

The present disclosure relates to the field of detection technology, and in particular to a tomosynthesis imaging apparatus and method for a target region.

### BACKGROUND

Digital tomosynthesis (DT) is a type of X-ray imaging method. Its characteristic is that only a small number of projection angles are required to obtain tomographic images parallel to a detector direction, thereby allowing acquisition of depth information inside an object. Traditional digital radiography (DR) produces superimposed images of object structures, and easily leads to misdiagnosis when a lesion is overlapped. In contrast, DT may acquire internal depth information of the object and improve diagnostic accuracy. Compared with traditional CT, DT has advantages of lower radiation dose and higher resolution.

Accordingly, there is a need to develop a digital tomosynthesis apparatus that is easy to operate and has a relatively simple structure.

### SUMMARY

According to an aspect of the present disclosure, a tomosynthesis imaging apparatus for a target region is provided, including: a distributed multi-point emission radiation source having an array of a plurality of point sources, where the plurality of point sources are configured to sequentially emit radiation beams; and a detector configured to be placed inside the target region or outside and adjacent to the target region to detect the radiation beams emitted by the distributed multi-point emission radiation source; where the detector is configured to start an acquisition of the radiation beams upon a detection of an increase in an intensity of the radiation beams, and to stop the acquisition upon a detection of a decrease in the intensity of the radiation beams.

In an embodiment, the target region is an oral cavity, and the detector is placed inside the oral cavity.

In an embodiment, the detector is configured to start the acquisition of the radiation beams upon a detection that the intensity of the radiation beams exceeds a first threshold.

In an embodiment, the detector is configured to stop the acquisition upon a detection that the intensity of the radiation beams falls below a second threshold.

In an embodiment, the detector is configured with a continuous multiple acquisition mode such that the detector repeatedly starts the acquisition of the radiation beams when the intensity of the radiation beams exceeds the first threshold and stops the acquisition when the intensity of the radiation beams falls below the second threshold to complete multiple acquisitions; or the detector is configured with an automatic multiple acquisition mode such that the detector starts the acquisition of the radiation beams upon a first detection that the intensity of the radiation beams exceeds the first threshold and stops the acquisition upon a first detection that the intensity of the radiation beams falls below the second threshold to complete a first acquisition, subsequently starts the acquisition of the radiation beams upon a second detection that the intensity of the radiation beams exceeds the first threshold, and automatically repeats multiple acquisitions according to a start time of the first acquisition, an acquisition duration, and a time interval between the start time of the first acquisition and a start time of the second acquisition; or the detector is configured with a predetermined multiple acquisition mode such that the detector starts the acquisition of the radiation beams upon a first detection that the intensity of the radiation beams exceeds the first threshold, and automatically performs subsequent acquisitions of the radiation beams according to a predetermined acquisition duration and a predetermined acquisition interval, where the predetermined acquisition duration and the predetermined acquisition interval are determined by the plurality of point sources of the distributed multi-point emission radiation source.

In an embodiment, the detector is configured to construct an image information based on the acquisition and output the image information in a wireless or wired manner.

In an embodiment, each of the plurality of point sources of the distributed multi-point emission radiation source is configured to emit an X-ray beam.

In an embodiment, the X-ray beams emitted by the plurality of point sources of the distributed multi-point emission radiation source have identical intensity and beam shape.

In an embodiment, the intensity of the radiation beams is greater than the first threshold and remains substantially constant during the acquisition.

In an embodiment, the target region is a human joint, a heart, or another body part.

In an aspect of the present disclosure, a tomosynthesis imaging method for a target region is provided, including: placing a detector at the target region to detect radiation beams; and emitting radiation beams toward an oral cavity from a plurality of positions; where the detector is configured to start an acquisition of the radiation beams upon a detection of an increase in an intensity of the radiation beams, and to stop the acquisition upon a detection of a decrease in the intensity of the radiation beams.

In an embodiment, the tomosynthesis imaging method further includes: starting the acquisition of the radiation beams upon a detection that the intensity of the radiation beams exceeds a first threshold.

In an embodiment, the tomosynthesis imaging method further includes: repeatedly starting the acquisition of the radiation beams when the intensity of the radiation beams exceeds the first threshold and stopping the acquisition when the intensity of the radiation beams falls below the second threshold to complete multiple acquisitions; or starting the acquisition of the radiation beams upon a first detection that the intensity of the radiation beams exceeds the first threshold and stopping the acquisition upon a first detection that the intensity of the radiation beams falls below the second threshold to complete a first acquisition, subsequently starting the acquisition of the radiation beams upon a second detection that the intensity of the radiation beams exceeds the first threshold, and automatically repeating multiple acquisitions according to a start time of the first acquisition, an acquisition duration, and a time interval between the start time of the first acquisition and a start time of the second acquisition; or starting the acquisition of the radiation beams upon a first detection that the intensity of the radiation beams exceeds the first threshold, and automatically performing subsequent acquisitions of the radiation beams according to a predetermined acquisition duration and a predetermined acquisition interval, where the predetermined acquisition duration and the predetermined acquisition interval are determined by a plurality of point sources of a distributed multi-point emission radiation source.

In an embodiment, the emitted radiation beams are X-ray beams.

In an embodiment, the intensity of the radiation beam emitted from each of the plurality of positions toward the oral cavity is greater than the first threshold.

In an embodiment, the tomosynthesis imaging method further includes: constructing an image information based on the acquisition, and outputting the image information in a wireless or wired manner.

In an embodiment, the radiation beams emitted from the plurality of positions toward the oral cavity have identical intensity and beam shape.

In an embodiment, the radiation beams emitted from the plurality of positions toward the oral cavity have substantially constant intensity and beam shape.

It should be understood that the content described in this section is not intended to identify key or important features of embodiments of the present disclosure, nor is it used to limit the scope of the present disclosure. Other features of the present disclosure will become easily understood through the following description.

### BRIEF DESCRIPTION OF THE DRAWINGS

The accompanying drawings are provided for better understanding of the solutions and are not intended to limit the present disclosure. In the accompanying drawings:
FIG. 1 shows an arrangement of a distributed multi-point emission radiation source and a detector according to an embodiment of the present disclosure;
FIG. 2 shows a first support according to an embodiment of the present disclosure;
FIG. 3 shows a second support according to an embodiment of the present disclosure; and
FIG. 4 shows a schematic diagram of the variation of an intensity of radiation beams over time according to an embodiment of the present disclosure.

### DETAILED DESCRIPTION OF EMBODIMENTS

To more clearly elaborate on the objectives, technical solutions, and advantages of the present disclosure, the embodiments of the present disclosure will be described in detail below with reference to the accompanying drawings. It should be understood that the following description of the embodiments is intended to explain and illustrate the general concept of the present disclosure, and should not be construed as limiting the present disclosure. In the specification and the accompanying drawings, the same or similar reference numerals denote the same or similar components or elements. For the sake of clarity, the drawings are not necessarily drawn to scale, and some well-known components and structures may be omitted from the drawings.

Unless otherwise defined, technical terms or scientific terms used in the present disclosure shall have the usual meanings understood by those of ordinary skilled in the field to which the present disclosure belongs. The terms such as "first", "second" and similar words used in the present disclosure do not indicate any order, quantity, or importance, but are merely used to distinguish different components. The term "a" or "an" does not exclude a plurality. The terms "including", "containing" or similar words mean that an element or item preceding the term encompasses the element or item listed after the term and their equivalents, but does not exclude other elements or items. The terms "connect", "couple" or similar words are not limited to physical or mechanical connections, but may include electrical connections, whether direct or indirect. The terms such as "upper", "lower", "left", "right", "top", or "bottom" are merely used to indicate relative positional relationships, and when the absolute position of the described object changes, the relative positional relationships may also change accordingly. When an element such as a layer, film, region, or substrate is referred to as being "on" or "under" another element, the element may be "directly" on or under the other element, or there may be an intermediate element therebetween.

In a tomographic imaging system, a radiation source and a detector cooperate to operate synchronously, that is, the radiation source emits radiation beams while the detector acquires the radiation beams transmitted through a target, thereby completing scanning and imaging of the target. In a tomosynthesis imaging method and apparatus for a target region, the detector is placed inside an oral cavity, for example behind gums, so that the radiation beams emitted from the radiation source pass through the tooth and received by the detector, thereby completing an imaging of the tooth. In existing systems or apparatuses, a controller or processor is provided to control emission and cessation of the radiation source. For example, the radiation source may include a high-voltage device to control, for example, a radiation source generation device, and the controller needs to control a voltage and current of the high-voltage device to generate radiation. The controller further controls a detection and signal acquisition of the detector, as well as image signal processing. Therefore, the controller or processor is connected to the detector in a wired or wireless manner to transmit control signals and image signals.

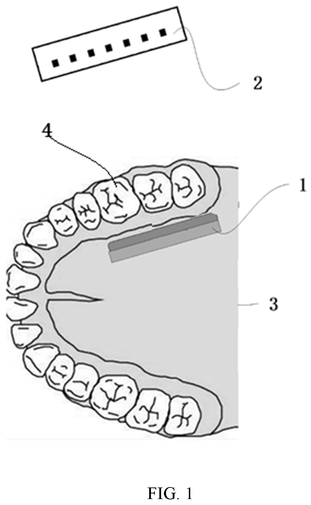

In an embodiment of the present disclosure, a tomosynthesis imaging apparatus is provided, in which the radiation source is a distributed multi-point emission radiation source 2 in order to obtain tomosynthesis imaging. The distributed multi-point emission radiation source 2 includes a plurality of point sources (black dots in FIG. 1) arranged in an array, and the plurality of point sources are configured to sequentially emit radiation beams. In an embodiment, the distributed multi-point emission radiation source 2 includes, for example, five point sources or seven point sources. In other embodiments, the distributed multi-point emission radiation source 2 may include other numbers of point sources, which may be set as needed by those skilled in the art according to the present disclosure.

In an embodiment of the present disclosure, the tomosynthesis imaging apparatus further includes a detector 1, which may be placed inside an oral cavity to detect the radiation beams emitted by the distributed multi-point emission radiation source 2. Generally, the detector 1 is placed inside the oral cavity in contact with a tooth 4. Because the detector 1 is placed inside the oral cavity 3, the detector 1 of the present disclosure has different configurations and functions from a conventional detector used for transmission imaging.

FIG. 1 shows an arrangement of the distributed multi-point emission radiation source 2 and the detector 1 according to an embodiment of the present disclosure. As shown in FIG. 1, the distributed multi-point emission radiation source 2 may be placed outside the oral cavity 3 and emit radiation beams toward a target region of the oral cavity 3. The detector 1 is placed inside the oral cavity, for example, on one side of part of the teeth 4, to receive radiation beams from the distributed multi-point emission radiation source 2 that have been transmitted through the tooth 4.

In an embodiment of the present disclosure, the distributed multi-point emission radiation source 2 is controlled by the tomosynthesis imaging apparatus so that, for example, seven point sources sequentially emit radiation beams to irradiate the tooth 4. In an embodiment, a scanning time for the plurality of point sources, such as seven point sources, to sequentially emit radiation beams once is very short, for example, 1~2 seconds. Therefore, any positional change of the detector 1 during this period is very small and negligible. Here, it should be noted that each point source of the distributed multi-point emission radiation source 2 may emit radiation, which is collimated or shaped into radiation beams by a collimator. In an embodiment, each point source may be provided with a separate collimator, or a single collimator may be provided for the entire distributed multi-point emission radiation source 2, so that radiation emitted by each point source is collimated or shaped into a desired radiation beam by the collimator. In the present disclosure, the description that a point source emits a radiation beam implicitly means that the radiation emitted by the point source is collimated into a desired radiation beam. Since the distributed multi-point emission radiation source 2 is arranged as in FIG. 1, the plurality of point sources are located at different positions relative to, for example, the same tooth 4. As shown in FIG. 1, the plurality of point sources are spatially displaced relative to the same tooth 4 in a lateral direction (left-right direction of the paper), so that orientations of the radiation beams emitted by the plurality of point sources to irradiate the same tooth 4 (i.e., irradiation angles on the tooth 4) are different from each other. In other words, the distributed multi-point emission radiation source 2 achieves irradiation of the same tooth 4 from multiple angles, thereby obtaining more cross-sectional information of the tooth 4 (through computed tomography calculation). By synthesizing the transmission images obtained from these different angles, a three-dimensional information of the tooth 4 may be obtained. The method for computing images through computed tomography may adopt methods known in the art.

In an embodiment of the present disclosure, the distributed multi-point emission radiation source 2 may be controlled such that the plurality of point sources sequentially emit radiation beams. For example, the tomosynthesis imaging apparatus includes a processor or controller that controls the plurality of point sources of the distributed multi-point emission radiation source 2 to sequentially emit radiation beams, including an order in which the plurality of point sources emit radiation beams, a start time and end time of radiation beam emission by each point source, and a time interval during which no radiation beam is emitted between two consecutive point sources that emit radiation beams.

In another embodiment of the present disclosure, the processor may also form part of the distributed multi-point emission radiation source 2. In other words, in this embodiment, the distributed multi-point emission radiation source 2 includes a processor or controller that controls the operation of the plurality of point sources, for example, parameters such as the start time and end time of radiation beam emission by each point source. That is to say, in this embodiment, the distributed multi-point emission radiation source 2 has the capability of sequentially emitting radiation beams, including parameters such as starting voltage as well as (start) emission time, emission duration, end time, and radiation intensity of each point source. In this embodiment, the tomosynthesis imaging apparatus includes the distributed multi-point emission radiation source 2 and the detector 1, and the emission time, emission duration, end time, radiation intensity and other parameters of the plurality of point sources of the distributed multi-point emission radiation source 2 may be preset or predetermined.

In an embodiment of the present disclosure, the detector 1 is placed inside the oral cavity and is capable of operating automatically. According to this embodiment, the detector 1 is a detector 1 operating in an automatic exposure mode, that is, it may automatically detect an energy or intensity of the radiation beams, start acquisition of the radiation beams upon a detection of an increase in the intensity of the radiation beams, and stop the acquisition upon a detection of a decrease in the intensity of the radiation beams. This is advantageous in that, since the detector 1 of the present disclosure operates in an automatic exposure mode, the detector does not need to be controlled by the processor or controller of the tomosynthesis imaging apparatus or by the processor or controller of the distributed multi-point emission radiation source 2 to detect and acquire the radiation beams. In other words, synchronization between the detector 1 and the distributed multi-point emission radiation source 2 of the present disclosure may be achieved without the need of a controller. Because the detector 1 does not need to be controlled by the processor or controller of the tomosynthesis imaging apparatus or of the distributed multi-point emission radiation source 2, there is no need to establish communication between the detector 1 and the controller through a wired connection, nor is there a need to establish wireless communication between the detector 1 and the processor or controller of the tomosynthesis imaging apparatus or of the distributed multi-point emission radiation source 2. Thus, no protocol is needed to achieve communication between the detector 1 and the controller to control the operation of the detector 1. This greatly simplifies the tomosynthesis imaging apparatus, improves the convenience of the detector 1, and also meet the requirement of synchronization between the emission of radiation beams by the distributed multi-point emission radiation source 2 and the detection and acquisition by the detector 1. In this embodiment, the configuration of the distributed multi-point emission radiation source, the detector placed inside the oral cavity that may automatically acquire signals according to the intensity or energy of radiation, and the absence of a physical connection between the distributed multi-point emission radiation source and the detector collectively achieves the effects of a simpler structure, easier operation, more convenient cleaning of the detector, and reduced manufacturing costs.

In the imaging process, embodiments of the present disclosure enable the detector to promptly detect and acquire radiation signals, and overcome the inertial thinking of those skilled in the art that the synchronization between the radiation source and the detector must be achieved by using a controller to control both simultaneously, for example, as in the prior art where a controller or processor of the radiation source controls both the radiation source and the detector. In embodiments of the present disclosure, the detector may operate in an automatic detection and acquisition mode, so that the detector starts operating when irradiated by the radiation beams. Further, the need of wired or wireless communication between the radiation source and the detector may be eliminated. Wired connections impose, for example, limitations on wiring and related structural configurations, and line aging and failure may prevent synchronization over time. Wireless connections require communication protocols, and detectors with different signals or different protocols need to be reset or customized, while during communication, external currents or electromagnetic waves may cause interference and deteriorate signal quality. The arrangement of the automatic acquisition detector of the present disclosure greatly improves operational convenience, solves the difficulty of inserting supports for holding detectors in some special positions, and further avoids the need for a controller to achieve the synchronization between the detector and the radiation source, thereby eliminating communication and related protocols. As a result, detectors of different models or from different manufacturers may be used without being affected by wiring limitations or aging problems caused by wired connections, and without being affected by external signal interference caused by wireless communication, thereby improving the operational convenience of the overall apparatus or system and enhancing a long-term stability.

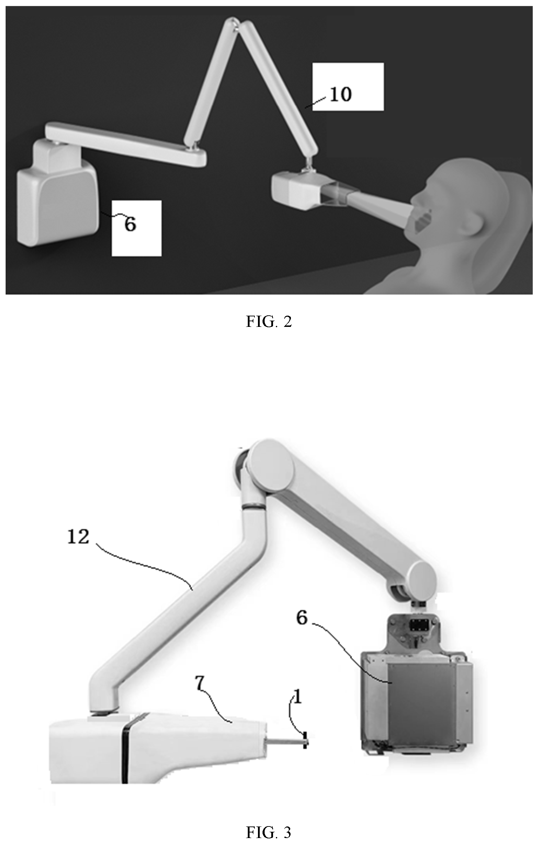

FIG. 2 shows a tomosynthesis imaging apparatus according to an embodiment of the present disclosure, in which the tomosynthesis imaging apparatus includes a first support 10. The first support 10 that is movable may support the distributed multi-point emission radiation source 2 and move the distributed multi-point emission radiation source 2 to a desired position, which greatly facilitates user operation. The first support 10 has a plurality of arm parts, which are connected to each other by universal joints, allowing the arm parts to move in three degrees of freedom (six directions including forward/backward, left/right, and up/down) and to rotate freely. In the embodiment shown in FIG. 2, the first support 10 is mechanically connected to, for example, a radiation source generator 6. In this embodiment, the detector 1 is not physically connected to the first support 10, or in other words, to the distributed multi-point emission radiation source 2. This is advantageous because such an arrangement allows the detector 1 to be easily placed inside the oral cavity 3 and removed from the oral cavity 3, and also facilitates cleaning of the detector 1 without any connecting devices. The first support 10 or the distributed multi-point emission radiation source 2 does not need to provide a connection interface with the detector 1, thus simplifying the structure.

FIG. 3 shows a tomosynthesis imaging apparatus according to an embodiment of the present disclosure, in which the tomosynthesis imaging apparatus includes a second support 11. The second support 11 that is movable may support the distributed multi-point emission radiation source 2 and move the distributed multi-point emission radiation source 2 to a desired position, which greatly facilitates user operation. In the embodiment shown in FIG. 3, the second support 11 is mechanically connected to, for example, a radiation source generator 6, and the radiation source generator may, for example, supply power to the distributed multi-point emission radiation source 2. The second support 11 has at least two arm parts, which are connected, for example, by two rotating shafts. The axes of the two rotating shafts are perpendicular to each other, allowing the two arm parts to move in three degrees of freedom (six directions including forward/backward, left/right, and up/down) and to rotate freely. In this embodiment, the detector 1 is physically connected to the second support 11, or in other words, to a housing 7 of the distributed multi-point emission radiation source 2. This is advantageous because such an arrangement allows the detector 1 to be easily placed inside the oral cavity 3 and to maintain a predetermined distance and orientation relative to a window of the housing 7 of the distributed multi-point emission radiation source 2. Since the detector 1 maintains a physical connection with the housing 7 of the distributed multi-point emission radiation source 2, whenever the detector 1 is placed inside the oral cavity, it does not need to be repositioned relative to the distributed multi-point emission radiation source 2. It should be noted that although the detector 1 is connected to the housing 7 of the distributed multi-point emission radiation source 2, the connection is purely mechanical in this embodiment, which only maintains the relative position between the detector 1 and the housing 7 of the distributed multi-point emission radiation source 2 and does not provide any wired or wireless communicative connection. In this embodiment, the detector 1 may be detached from the connection.

The first support 10 and the second support 11 represent different embodiments of the present disclosure and need not to be provided simultaneously.

According to an embodiment of the present disclosure, in the tomosynthesis imaging apparatus, the detector 1 is configured to start acquisition of the radiation beams upon a detection of an increase in the intensity of the radiation beams. For example, in the absence of radiation beams, the detector 1 may remain in a standby state, such as a state where it is powered on but not operating. When irradiation begins, the detector 1 detects an increase in the intensity of the radiation beams and starts operating to acquire signals of the radiation beams. When a sensitive element of the detector 1 is exposed to the radiation, the detector 1 starts detecting the radiation beams and acquiring the radiation beams. In an embodiment of the present disclosure, the detector 1 starts acquisition of the radiation beams upon a detection that the intensity of the radiation beams exceeds a first threshold. The first threshold may be set according to actual conditions. For example, the first threshold may be set as a specific value less than an intensity value of the radiation beams emitted by the point source of the distributed multi-point emission radiation source 2.

In an embodiment, the detector 1 is further configured to stop the acquisition of the radiation beams upon a detection of a decrease in the intensity value of the radiation beams. In an embodiment, the detector 1 is configured to stop the acquisition of the radiation beams upon a detection that the intensity of the radiation beams is less than a second threshold. The second threshold may be set according to actual conditions. For example, the second threshold may be set as a specific value less than the intensity value of the radiation beams emitted by the point source of the distributed multi-point emission radiation source 2. In embodiments of the present disclosure, the first threshold and the second threshold may be reasonably set according to actual conditions, and the two may be the same value or different values.

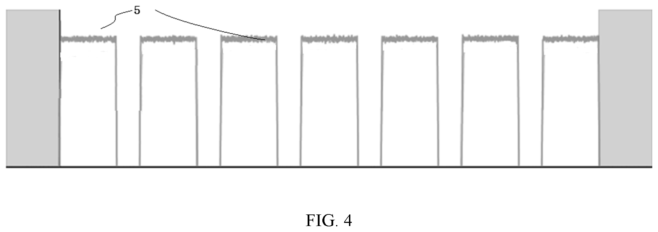

By setting the first threshold, the detector 1 may be prevented from being interfered by external factors, and may start acquiring the radiation beams only when, for example, irradiated by the radiation beams, thereby avoiding false operations. This ensures a high sensitivity of the detector 1 while preventing false operations caused by environmental radiation. For example, the plurality of point sources of the distributed multi-point emission radiation source 2 may sequentially emit radiation beams, and the energy of the radiation beams is schematically shown in FIG. 4. During the emission of radiation beams by each point source, the radiation beams emitted by the point source have a constant energy value 5. In FIG. 4, the horizontal axis may represent time and the vertical axis may represent energy value, with no units shown for simplicity. The first threshold is lower than the radiation beam energy 5 shown in FIG. 4. The radiation beam energy at a rising edge of an energy curve or intensity curve of the radiation beams may trigger the detector 1 to start operating, such as beginning acquisition of the energy of the radiation beams. Upon a detection that the energy of the radiation beams is less than the second threshold, the detector 1 stops the acquisition. As shown in FIG. 4, when the intensity curve of the radiation beams passes the second threshold on a falling edge, the detector 1 stops the acquisition. Thus, the detector 1 may ensure acquisition of the entire radiation beams, while remaining inactive when no radiation beam is present, thereby avoiding false operations due to noise or interference, which would otherwise cause measurement errors.

In an embodiment of the present disclosure, the radiation beam emitted by each point source of the distributed multi-point emission radiation source 2 has identical or substantially identical energy value or intensity value 5 and beam shape. FIG. 4 shows a form of the variation of the energy value or intensity value over time; however, it should be understood that other forms are also possible, and the rising edges and falling edges of the energy or intensity may have other forms. The beam shape of the radiation beam from the point source may be, for example, a rectangular radiation beam, a fan radiation beam, or a conical radiation beam, etc. Each point source of the distributed multi-point emission radiation source 2 may emit, for example, an X-ray beam; however, other types of point sources may also be employed.

The detector 1 may construct an image information based on the acquisition and output the image information in a wireless or wired manner.

In an embodiment of the present disclosure, the detector 1 is configured to support multiple acquisition modes.

In an embodiment, the detector 1 supports a continuous multiple acquisition mode, in which the detector 1 may repeatedly start acquisition of the radiation beams upon each detection that the intensity of the radiation beams exceeds the first threshold and stop the acquisition upon each detection that the intensity of the radiation beams falls below the second threshold. The detector 1 operates according to the intensity of the radiation beams during each cycle, thereby completing multiple acquisitions. This mode provides strong adaptability and may adapt to different irradiation requirements without specific settings.

In an embodiment, the detector 1 is configured to support an automatic multiple acquisition mode. In this mode, the detector 1 starts acquisition of the radiation beams upon a first detection that the intensity of the radiation beams exceeds the first threshold and stops the acquisition upon a first detection that the intensity of the radiation beams falls below the second threshold, thereby completing a first acquisition. Subsequently, the detector 1 starts acquisition of the radiation beams upon a second detection that the intensity of the radiation beams exceeds the first threshold. At this point, the detector 1 has already obtained a start time of the first acquisition, a start time of the second acquisition, and a time interval between the two. According to the known start time of the first acquisition, acquisition duration, and time interval between the start time of the first acquisition and the start time of the second acquisition, the detector 1 may automatically repeat multiple acquisitions. In this embodiment, the detector 1 may store the start time of the first acquisition and the start time of the second acquisition, and calculate the time interval between the start time of the first acquisition and the start time of the second acquisition.

In an embodiment, the detector 1 supports a predetermined multiple acquisition mode. In this mode, the detector 1 starts acquisition of the radiation beams upon a first detection that the intensity of the radiation beams exceeds the first threshold, and automatically performs subsequent acquisitions of the radiation beams according to a predetermined acquisition duration and a predetermined acquisition interval. In an embodiment, the predetermined acquisition duration and the predetermined acquisition interval are determined by the plurality of point sources of the distributed multi-point emission radiation source 2.

In an embodiment of the present disclosure, the detector 1 may be an independent component, which may include a built-in processor or controller to control the operation of the radiation-sensitive element, control the power supply, process electrical signals converted from radiation signals by the radiation-sensitive elements into image information, and so on.

In an aspect of the present disclosure, a tomosynthesis imaging apparatus is provided, which includes a radiation source and a detector. The radiation source is the distributed multi-point emission radiation source as described in the foregoing embodiments, and the detector may be the above-mentioned detector, which may start or stop signal acquisition according to the increase or decrease in the energy or intensity of the received radiation beams. In this embodiment, the tomosynthesis imaging apparatus is provided for multi-angle imaging of the target region to obtain an image with three-dimensional information. In this embodiment, for example, the distributed multi-point emission radiation source and the detector are placed on opposite sides of a human joint to irradiate the human joint from multiple angles and acquire radiation signals, thereby reconstructing a three-dimensional image of the human joint. In another embodiment of the present disclosure, the target region may be a heart, a liver, or other body parts, and a three-dimensional image may be obtained through the cooperation of the distributed multi-point emission radiation source and the detector. Since the detector of the present disclosure may operate without a physical connection or communicative connection with the distributed multi-point emission radiation source, it may be, for example, attached to a back of a human body or other suitable parts to perform CT imaging of the human lungs, which greatly improves the convenience of practical operation. For the distributed multi-point emission radiation source and the detector, reference may be made to the foregoing embodiments of the present disclosure.

In another aspect of the present disclosure, an intraoral tomosynthesis imaging method is provided, including: placing a detector 1 inside an oral cavity to detect radiation beams; and emitting radiation beams toward the oral cavity 3 from a plurality of positions. In this embodiment, the detector 1 is configured to start acquisition of the radiation beams upon a detection of an increase in an intensity of the radiation beams, and stop the acquisition upon a detection of a decrease in the intensity of the radiation beams.

In the method of the present disclosure, the radiation beams are emitted toward the oral cavity 3 from a plurality of positions, which enables irradiation on the same tooth 4 with the same radiation from different angles, thereby obtaining three-dimensional information of the tooth 4. For example, in an embodiment of the present disclosure, a distributed multi-point emission radiation source 2 is used, in which a plurality of point sources, such as seven point sources as shown in FIG. 1, sequentially emit radiation beams to irradiate the tooth 4. Each point source is located at a different position and thus irradiate the same tooth 4 from a different irradiation angle, thereby obtaining different irradiation information. By synthesizing the transmission images from different angles, tomosynthesis imaging is achieved to obtain the three-dimensional information of the tooth 4. The distributed multi-point emission radiation source 2 may be controlled such that the plurality of point sources sequentially emit radiation beams. For example, the intraoral tomosynthesis imaging apparatus or the distributed multi-point emission radiation source 2 includes a processor that controls the plurality of point sources of the distributed multi-point emission radiation source 2 to sequentially emit radiation beams, including an order in which the plurality of point sources emit radiation beams, the start time and end time of radiation beam emission by each point source, and a time interval during which no radiation beam is emitted between two consecutive point sources that emit radiation beams. In another embodiment of the present disclosure, the processor may also form part of the distributed multi-point emission radiation source 2. In other words, in this embodiment, the distributed multi-point emission radiation source 2 includes a processor that controls the operation of the plurality of point sources, for example, parameters such as the start time and end time of radiation beam emission by each point source. That is to say, in this embodiment, the distributed multi-point emission radiation source 2 has the capability of sequentially emitting radiation beams, including parameters such as the emission time, end time, and radiation beam energy of each point source.

In this embodiment, the detector 1 is placed inside the oral cavity and is capable of operating automatically. According to this embodiment, the detector 1 supports an automatic exposure mode, that is, it may automatically detect the energy or intensity of the radiation beams, start acquisition of the radiation beams when the intensity of the radiation beams increases, and stop the acquisition when the detected intensity of the radiation beams decreases. The processor of the intraoral tomosynthesis imaging apparatus or of the distributed multi-point emission radiation source 2 does not control the operation of the detector 1, nor does the processor have any communicative connection or even physical connection with the detector 1. In this embodiment, it is unnecessary to control the detector 1 to start detecting the radiation beams or to start acquiring radiation beam signals, and the detector 1 may automatically detect the increase in the intensity of the radiation beams and start detecting and acquiring the radiation beams. According to an embodiment of the present disclosure, the detector 1 is configured to start the acquisition of the radiation beams upon a detection of an increase in the intensity of the radiation beams. For example, in the absence of radiation beams, the detector 1 may remain in a standby state, such as a state where it is powered on but not operating. When irradiation begins, the detector 1 detects an increase in the intensity of the radiation beams and starts operating to acquire signals of the radiation beams. When a sensitive element of the detector 1 is exposed to the radiation, the detector 1 starts detecting the radiation beams and acquiring the radiation beams. For example, the detector 1 starts acquisition of the radiation beams upon a detection that the intensity of the radiation beams exceeds a first threshold. The first threshold may be set according to actual conditions. For example, the first threshold may be set as a specific value less than an intensity value of the radiation beams emitted by the point source of the distributed multi-point emission radiation source 2. The detector 1 stops acquisition of the radiation beams upon a detection of a decrease in the intensity value of the radiation beams. In an embodiment, the detector 1 is configured to stop the acquisition of the radiation beams upon a detection that the intensity of the radiation beams falls below a second threshold. The second threshold may be set according to actual conditions. For example, the second threshold may be set as a specific value less than the intensity value of the radiation beams emitted by the point sources of the distributed multi-point emission radiation source 2. In embodiments of the present disclosure, the first threshold and the second threshold may be reasonably set according to actual conditions, and the two may be the same or different.

The radiation beams may be emitted by the plurality of point sources of the distributed multi-point emission radiation source 2, and the radiation beam emitted by each point source has identical or substantially identical energy or intensity and beam shape. The beam shape of the radiation beams may be, for example, a rectangular radiation beam, a fan radiation beam, or a conical radiation beam, etc. The radiation beam may be, for example, an X-ray beam; however, other types of radiation are also possible. In an embodiment of the present disclosure, the radiation beams emitted toward the oral cavity 3 from the plurality of positions each maintain a constant or substantially constant intensity, the intensity of the radiation beams is known, and the intensity of the radiation beams is greater than the first threshold, thereby obtaining tomographic images of the irradiated tooth 4 from multiple angles, which are then synthesized into a three-dimensional image through computer calculation.

An image information may be constructed based on the acquisition, and the image information is output in a wireless or wired manner.

In an embodiment of the present disclosure, the method includes: starting the detector 1 to acquire the radiation beams each time the intensity of the radiation beams exceeds the first threshold, and stopping the acquisition when the intensity of the radiation beams falls below the first threshold, thereby performing multiple acquisitions. The method of this embodiment may correspond to a continuous multiple acquisition mode, which has strong adaptability and may adapt to different irradiation requirements without specific settings.

In another embodiment of the present disclosure, the method includes: starting acquisition of the radiation beams upon a first detection that the intensity of the radiation beams exceeds the first threshold and stopping the acquisition upon a second detection that the intensity of the radiation beams falls below the first threshold, to complete a first acquisition; subsequently starting acquisition of the radiation beams again when the intensity of the radiation beams exceeds the first threshold; and automatically repeating multiple acquisitions according to the known parameters including the start time of the first acquisition, acquisition duration, and time interval between the start time of the first acquisition and the start time of the second acquisition. The method of this embodiment may correspond to an automatic multiple acquisition mode, in which the detector 1 may store the start time of the first acquisition and the start time of the second acquisition, and calculate the time interval between the start time of the first acquisition and the start time of the second acquisition.

In another embodiment of the present disclosure, the method includes: starting acquisition of the radiation beams upon a first detection that the intensity of the radiation beams exceeds the first threshold, and automatically performing subsequent acquisitions of the radiation beams according to a predetermined acquisition duration and a predetermined acquisition interval. In this embodiment, the predetermined acquisition duration and the predetermined acquisition interval are determined by the plurality of point sources of the distributed multi-point emission radiation source 2.

It should be understood that the various forms of processes shown above may be reordered, with steps added or removed. For example, the steps described in the present disclosure may be performed in parallel, sequentially, or in a different order, as long as the desired results of the technical solution in the present disclosure may be achieved. No limitation is imposed herein.

The foregoing specific embodiments do not constitute limitations on the scope of protection of the present disclosure. It should be understood by those skilled in the art that various modifications, combinations, sub-combinations, and substitutions may be made according to design requirements and other factors. Any modifications, equivalent substitutions, improvements, etc., made within the spirit and principles of the present disclosure should fall within the scope of protection of the present disclosure.

## Claims

1. A tomosynthesis imaging apparatus for a target region, comprising:
a distributed multi-point emission radiation source having an array of a plurality of point sources, wherein the plurality of point sources are configured to sequentially emit radiation beams; and
a detector configured to be placed inside the target region or outside and adjacent to the target region to detect the radiation beams emitted by the distributed multi-point emission radiation source;
wherein the detector is configured to start an acquisition of the radiation beams upon a detection of an increase in an intensity of the radiation beams, and to stop the acquisition upon a detection of a decrease in the intensity of the radiation beams.

2. The tomosynthesis imaging apparatus of claim 1, wherein the target region is an oral cavity, and the detector is placed inside the oral cavity.

3. The tomosynthesis imaging apparatus of claim 1, wherein the detector is configured to start the acquisition of the radiation beams upon a detection that the intensity of the radiation beams exceeds a first threshold.

4. The tomosynthesis imaging apparatus of claim 3, wherein the detector is configured to stop the acquisition upon a detection that the intensity of the radiation beams falls below a second threshold.

5. The tomosynthesis imaging apparatus of claim 4, wherein:
the detector is configured with a continuous multiple acquisition mode such that the detector repeatedly starts the acquisition of the radiation beams when the intensity of the radiation beams exceeds the first threshold and stops the acquisition when the intensity of the radiation beams falls below the second threshold to complete multiple acquisitions; or
the detector is configured with an automatic multiple acquisition mode such that the detector starts the acquisition of the radiation beams upon a first detection that the intensity of the radiation beams exceeds the first threshold and stops the acquisition upon a first detection that the intensity of the radiation beams falls below the second threshold to complete a first acquisition, subsequently starts the acquisition of the radiation beams upon a second detection that the intensity of the radiation beams exceeds the first threshold, and automatically repeats multiple acquisitions according to a start time of the first acquisition, an acquisition duration, and a time interval between the start time of the first acquisition and a start time of the second acquisition; or
the detector is configured with a predetermined multiple acquisition mode such that the detector starts the acquisition of the radiation beams upon a first detection that the intensity of the radiation beams exceeds the first threshold, and automatically performs subsequent acquisitions of the radiation beams according to a predetermined acquisition duration and a predetermined acquisition interval, wherein the predetermined acquisition duration and the predetermined acquisition interval are determined by the plurality of point sources of the distributed multi-point emission radiation source.

6. The tomosynthesis imaging apparatus of claim 1, wherein the detector is configured to construct an image information based on the acquisition and output the image information in a wireless or wired manner.

7. The tomosynthesis imaging apparatus of claim 1, wherein each of the plurality of point sources of the distributed multi-point emission radiation source is configured to emit an X-ray beam.

8. The tomosynthesis imaging apparatus of claim 7, wherein the X-ray beams emitted by the plurality of point sources of the distributed multi-point emission radiation source have identical intensity and beam shape.

9. The tomosynthesis imaging apparatus of claim 3, wherein the intensity of the radiation beams is greater than the first threshold and remains substantially constant during the acquisition.

10. The tomosynthesis imaging apparatus of claim 1, wherein the target region is a human joint, a heart, or another body part.

11. A tomosynthesis imaging method for a target region, comprising:
placing a detector at the target region to detect radiation beams; and
emitting radiation beams toward an oral cavity from a plurality of positions;
wherein the detector is configured to start an acquisition of the radiation beams upon a detection of an increase in an intensity of the radiation beams, and to stop the acquisition upon a detection of a decrease in the intensity of the radiation beams.

12. The tomosynthesis imaging method of claim 11, further comprising:
starting the acquisition of the radiation beams upon a detection that the intensity of the radiation beams exceeds a first threshold.

13. The tomosynthesis imaging method of claim 12, further comprising:
repeatedly starting the acquisition of the radiation beams when the intensity of the radiation beams exceeds the first threshold and stopping the acquisition when the intensity of the radiation beams falls below a second threshold to complete multiple acquisitions; or
starting the acquisition of the radiation beams upon a first detection that the intensity of the radiation beams exceeds the first threshold and stopping the acquisition upon a first detection that the intensity of the radiation beams falls below the second threshold to complete a first acquisition, subsequently starting the acquisition of the radiation beams upon a second detection that the intensity of the radiation beams exceeds the first threshold, and automatically repeating multiple acquisitions according to a start time of the first acquisition, an acquisition duration, and a time interval between the start time of the first acquisition and a start time of the second acquisition; or
starting the acquisition of the radiation beams upon a first detection that the intensity of the radiation beams exceeds the first threshold, and automatically performing subsequent acquisitions of the radiation beams according to a predetermined acquisition duration and a predetermined acquisition interval, wherein the predetermined acquisition duration and the predetermined acquisition interval are determined by a plurality of point sources of a distributed multi-point emission radiation source.

14. The tomosynthesis imaging method of claim 11, wherein the emitted radiation beams are X-ray beams.

15. The tomosynthesis imaging method of claim 12, wherein the intensity of the radiation beam emitted from each of the plurality of positions toward the oral cavity is greater than the first threshold.

16. The tomosynthesis imaging method of claim 11, further comprising:
constructing an image information based on the acquisition, and outputting the image information in a wireless or wired manner.

17. The tomosynthesis imaging method of claim 15, wherein the radiation beams emitted from the plurality of positions toward the oral cavity have identical intensity and beam shape.

18. The tomosynthesis imaging method of claim 15, wherein the radiation beams emitted from the plurality of positions toward the oral cavity have substantially constant intensity and beam shape.
